# EUROPÄISCHE PATENTSCHRIFT

(11) **EP 1 654 061 B1**
(45) Veröffentlichungstag und Bekanntmachung des Hinweises auf die Patenterteilung: **25.04.2007**
(21) Anmeldenummer: 04763675.8
(22) Anmeldetag: 30.07.2004
(51) Int. Cl.: B01J 31/06, C07C 51/215

(54) **KATALYSATOR FÜR GASPHASENOXIDATIONEN**
CATALYST FOR GAS PHASE OXIDATIONS
CATALYSEUR POUR OXYDATIONS EN PHASE GAZEUSE

(30) Priorität: 01.08.2003 DE 10335346
(43) Veröffentlichungstag der Anmeldung: 10.05.2006
(73) Patentinhaber: BASF Aktiengesellschaft, 67056 Ludwigshafen (DE)
(72) Erfinder: NETO, Samuel, 01099 Dresden (DE); ZÜHLKE, Jürgen, 67346 Speyer (DE); STORCK, Sebastian, 68167 Mannheim (DE); ROSOWSKI, Frank, 68165 Mannheim (DE)
(74) Vertreter: Reitstötter - Kinzebach
(86) Internationale Anmeldenummer: PCT/EP2004/008596
(87) Internationale Veröffentlichungsnummer: WO 2005/011862

(56) Entgegenhaltungen:
- WO-A-99/61433
- DE-A1- 19 824 532
- US-B1- 6 586 361

## Beschreibung

Die Erfindung betrifft einen Katalysator für Gasphasenoxidationen, der einen inerten Träger und eine unter Zuhilfenahme eines polymeren Bindemittels darauf aufgebrachte, Übergangsmetalloxide enthaltende katalytisch aktive Masse umfasst, sowie ein Verfahren zur katalytischen Gasphasenoxidation von aromatischen Kohlenwasserstoffen zu Carbonsäuren und/oder Carbonsäureanhydriden unter Verwendung des Katalysators.

Eine Vielzahl von Carbonsäuren und/oder Carbonsäureanhydriden wird technisch durch die katalytische Gasphasenoxidation von aromatischen Kohlenwasserstoffen, wie Benzol, den Xylolen, Naphthalin, Toluol oder Durol, in Festbettreaktoren, vorzugsweise Rohrbündelreaktoren, hergestellt. Man kann auf diese Weise z. B. Benzoesäure, Maleinsäureanhydrid, Phthalsäureanhydrid, Isophthalsäure, Terephthalsäure oder Pyromellithsäureanhydrid erhalten. Im Allgemeinen leitet man ein Gemisch aus einem molekularen Sauerstoff enthaltenden Gas, beispielsweise Luft und dem zu oxidierenden Ausgangsmaterial durch eine Vielzahl in einem Reaktor angeordneter Rohre, in denen sich eine Schüttung mindestens eines Katalysators befindet. Zur Temperaturregelung sind die Rohre von einem Wärmeträgermedium, beispielsweise einer Salzschmelze, umgeben.

Als Katalysatoren haben sich für diese Oxidationsreaktionen so genannte Schalenkatalysatoren bewährt, bei denen die katalytisch aktive Masse schalenförmig auf einem inerten Trägermaterial, wie Steatit aufgebracht ist. Als katalytisch aktiver Bestandteil der katalytisch aktiven Masse dieser Schalenkatalysatoren dient im allgemeinen neben Titandioxid (in Form seiner Anatasmodifikation) Vanadiumpentoxid. Des weiteren können in der katalytisch aktiven Masse in geringen Mengen eine Vielzahl anderer oxidischer Verbindungen enthalten sein, die als Promotoren die Aktivität und Selektivität des Katalysators beeinflussen.

Zur Herstellung derartiger Schalenkatalysatoren wird eine wässrige und/oder ein organisches Lösungsmittel enthaltende Lösung oder Suspension der Aktivmassenbestandteile und/oder deren Vorläuferverbindungen auf das Trägermaterial bei erhöhter Temperatur aufgesprüht, bis der gewünschte Aktivmassenanteil am Katalysatorgesamtgewicht erreicht ist.

Um die Qualität der Beschichtung zu verbessern, wurde in der Technik dazu übergegangen, der Suspension organische Binder, bevorzugt Copolymere, vorteilhaft in Form einer wässrigen Dispersion, von Vinylacetat/Vinyllaurat, Vinylacetat/Acrylat, Styrol/Acrylat sowie Vinylacetat/Ethylen zuzusetzen. Der Binderzusatz hat zudem den Vorteil, dass die Aktivmasse gut auf dem Träger haftet, so dass Transport und Einfüllen des Katalysators erleichtert werden.

Bei der thermischen Behandlung bei Temperaturen über 200 bis 500 °C entweicht der Binder durch thermische Zersetzung und/oder Verbrennung aus der aufgetragenen Schicht. Meist erfolgt die thermische Behandlung in situ im Oxidationsreaktor.

Aus der EP-A 0 744 214 ist ein Trägerkatalysator bekannt, der durch Aufbringen einer Oberflächenbeschichtung auf einen inerten Trägerkörper erhalten wird. Als organische Binder sind Vinylacetat/Vinyllaurat, Vinylacetat/Acrylat, Styrol/Acrylat, Vinylacetat/Maleat sowie Vinylacetat/Ethylen genannt.

Die DE-A 197 17 344 beschreibt ein Verfahren zur Herstellung von Katalysatoren, bei dem ein Gemisch von Oxiden in Gegenwart von Wasser gemahlen und anschließend auf Trägerkörper aufgetragen wird. Als organische Binder sind Vinylacetat/Vinyllaurat, Vinylacetat/Acrylat, Styrol/Acrylat, Vinylacetat/Maleat sowie Vinylacetat/Ethylen genannt.

Die US-A 4,397,768 beschreibt einen Katalysator zur Phthalsäureanhydridherstellung. Die aktive Masse wird mittels organischer Binder wie Vinylacetat/Vinyllaurat, Vinylacetat/Acrylat, Styrol/Acrylat, Vinylacetat/Maleat oder Vinylacetat/Ethylen auf einen inerten Träger aufgebracht.

Aus der DE-A 198 24 532 ist ein Bindemittel zur Herstellung von Schalenkatalysatoren bekannt, das aus einem Polymerisat von ethylenisch ungesättigten Säureanhydriden und einem Alkanolamin mit mindestens 2 OH-Gruppen, höchstens 2 Stickstoffatomen und höchstens 8 C-Atomen besteht.

Die EP-A 0 068 192 beschreibt ein Verfahren zur Herstellung abriebfester Schalenkatalysatoren. Als Bindemittel werden Glucose oder Harnstoff empfohlen.

Aus der DE-A 22 38 067 geht ein Trägerkatalysator hervor, dessen V₂O₅/TiO₂ enthaltende Aktivmasse mittels einer Vinylacetat-Vinyllaurat-Copolymerdispersion mit 25 Gew.-% Vinyllaurat auf Trägerkörper aufgebracht wird. Vinyllaurat-haltige Copolymerdispersionen sind Spezialdispersionen, die nicht in großer Menge verfügbar sind und deren Einsatz die Rohstoffkosten des Katalysators verteuert.

Der Erfindung liegt die Aufgabe zu Grunde, einen Katalysator für Gasphasenoxidationen bereitzustellen, der unter Verwendung handelsüblicher Copolymerdispersionen hergestellt ist und eine hohe Aktivität bezüglich der zu katalysierenden Gasphasenoxidation aufweist.

Es wurde nun gefunden, dass die Monomerzusammensetzung des polymeren Bindemittels einen bedeutenden Einfluss auf die Aktivität des erhaltenen Schalenkatalysators hat.

Die Erfindung betrifft einen Katalysator für Gasphasenoxidationen, der einen inerten Träger und eine darauf aufgebrachte, Übergangsmetalloxide enthaltende katalytisch aktive Masse umfasst, oder einen Präkatalysator für den Katalysator, wobei der (Prä)Katalysator erhältlich ist durch Behandeln des inerten Trägers mit einer wässrigen Suspension oder Lösung der Übergangsmetalloxide oder deren Vorläuferverbindungen, die außerdem eine Bindemitteldispersion enthält, wobei das Bindemittel ein Copolymer eines α-Olefins und eines Vinyl-C₂-C₄-carboxylats ist, dessen Vinyl-C₂-C₄-carboxylatgehalt wenigstens 62 Mol-%, vorzugsweise 63 bis 95 Mol%, beträgt.

Die Ursachen für den Einfluss des Bindemittels auf die Aktivität des erhaltenen Katalysators sind nicht vollständig aufgeklärt. Katalysatoren für Gasphasenoxidationen enthalten redoxaktive Übergangsmetalloxide wie V₂O₅. Als katalytische Zentren kommen hierbei Vandylgruppen (V=O) oder V-O-V- oder V-O-Träger-Brücken in Betracht. Nach Grzybowska [B. Grzybowska-Swierkosz, "Vanadia-Titania Catalysts for Oxidation of o-Xylene and other Hydrocarbons", Appl. Catal. A: General 157 (1997) 263-310] ist Vanadylgruppen zwar die Fähigkeit zur Wasserstoffabstraktion zuzuordnen, der Sauerstoff ist jedoch sehr stark gebunden, so dass keine Insertion des Sauerstoffatoms in eine Kohlenstoff-Wasserstoff-Bindung des zu oxidierenden Substrats möglich ist. V-O-V- oder V-O-Träger-Gruppen besitzen hingegen die Fähigkeit zur Sauerstoffinsertion. Nach Went et al. [G. T. Went, L.-J. Leu, A. T. Bell, "Quantitative Structural Analysis of Dispersed Vanadia Species in TiO₂ (Anatase)-Supported V₂O₅" J. Catal. 134 (1992) 479-491] werden endständige Sauerstoffatome leichter von H₂ reduziert. Wie in den nachfolgenden Beispielen gezeigt, weisen die erfindungsgemäßen Katalysatoren bei der temperaturprogrammierten Reduktion (TPR) eine verringerte H₂-Aufnahme auf; offenbar ist der Anteil monomerer Vanadylspezies (mit V=O Gruppen) zugunsten polymerer Vanadylspezies verringert. Vermutlich kommt es zu einer Komplexierung der Übergangsmetallspezies durch das erfindungsgemäß eingesetzte Bindemittel, wodurch die Art und Weise der Abscheidung auf dem Träger modifiziert wird.

Erfindungsgemäß verwendet man als Bindemittel ein Copolymer eines α-Olefins und eines Vinyl-C₂-C₄-carboxylats mit hohem Vinyl-C₂-C₄-carboxylatgehalt (Der "Gehalt" eines Copolymers an einem bestimmten Monomer bezieht sich auf den Gehalt an einpolymerisierten Einheiten des Monomers). Bei den Copolymeren handelt es sich in der Regel um statistische Copolymere. Geeignete Vinyl-C₂-C₄-carboxylate sind vor allem Vinylacetat und Vinylpropionat, wovon Vinylacetat besonders bevorzugt ist. Als Comonomere kommen α-Olefine mit 2 bis 20 Kohlenstoffatomen, insbesondere Ethylen, Propylen, Buten, Hexen oder Octen, in Betracht, wovon Ethylen bevorzugt ist. Ethylen-Vinylacetat-Copolymere sind besonders bevorzugt, insbesondere solche mit 63 bis 70 Mol-% Vinylacetat und 37 bis 30 Mol-% Ethylen.

Die erfindungsgemäß eingesetzten Bindemittel sind als wässrige Dispersionen handelsüblich, mit einem Feststoffgehalt von z. B. 35 bis 65 Gew.-%. Die eingesetzte Menge solcher Bindemitteldispersionen beträgt im Allgemeinen 2 bis 45 Gew.-%, vorzugsweise 5 bis 35 Gew.-%, besonders bevorzugt 7 bis 20 Gew.-%, bezogen auf das Gewicht der Lösung oder Suspension der Übergangsmetalloxide oder deren Vorläuferverbindungen. Der Gehalt gelöster und/oder suspendierter Übergangsmetalloxide oder deren Vorläuferverbindungen in der Lösung oder Suspension beträgt dabei im Allgemeinen 20 bis 50 Gew.-%.

Vorzugsweise enthält die katalytisch aktive Masse im calzinierten Zustand, bezogen auf die Gesamtmenge der katalytisch aktiven Masse, 1 bis 40 Gew.-% Vanadiumoxid, berechnet als V₂O₅, und 60 bis 99 Gew.-% Titandoxid, berechnet als TiO₂. Die katalytisch aktive Masse kann daneben bis zu 1 Gew.-% einer Cäsiumverbindung, berechnet als Cs, bis zu 1 Gew.-% einer Phosphorverbindung, berechnet als P und bis zu 10 Gew.-% Antimonoxid, berechnet als Sb₂O₃ enthalten.

Neben den fakultativen an Zusätzen Cäsium und Phosphor können im Prinzip in der katalytisch aktiven Masse in geringen Mengen eine Vielzahl anderer oxidischer Verbindungen enthalten sein, die als Promotoren die Aktivität und Selektivität des Katalysators beeinflussen, beispielsweise indem sie seine Aktivität absenken oder erhöhen. Als solche Promotoren seien beispielhaft die Alkalimetalloxide, insbesondere außer dem genannten Cäsiumoxid, Lithium-, Kalium- und Rubidiumoxid, Thallium(I)oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid genannt. In der Regel wird aus dieser Gruppe Cäsium als Promotor verwendet.

Ferner kommen von den genannten Promotoren noch bevorzugt als Zusätze die Oxide von Niob und Wolfram in Mengen von 0,01 bis 0,50 Gew.-%, bezogen auf die katalytisch wirksame Masse in Betracht. Als die Aktivität erhöhenden aber die Selektivität vermindernden Zusatz kommen vor allem oxidische Phosphorverbindungen insbesondere Phosphorpentoxid in Betracht.

Die katalytisch aktive Masse kann auch in zwei oder mehreren Schichten aufgebracht sein, wobei z. B. die innere Schicht oder die innere Schichten einen Antimonoxidgehalt von bis zu 15 Gew.-% und die äußere Schicht einen um 50 bis 100% verringerten Antimonoxidgehalt aufweisen. Dabei ist in der Regel die innere Schicht des Katalysators phosphorhaltig und die äußere Schicht phosphorarm oder phosphorfrei.

Die Schichtdicke der katalytisch aktiven Masse beträgt in der Regel 0,02 bis 0,2 mm, vorzugsweise 0,05 bis 0,15 mm. Der Aktivmasseanteil am Katalysator beträgt üblicherweise 5 bis 25 Gew.-%, meist 7 bis 15 Gew.-%.

Das eingesetzte Titandioxid besteht vorteilhaft aus einem Gemisch eines TiO₂ mit einer BET-Oberfläche von 5 bis 15 m²/g und eines TiO₂ mit einer BET-Oberfläche von 15 bis 50 m²/g. Man kann auch ein Titandioxid mit einer BET-Oberfläche von 5 bis 50 m/g, vorzugsweise 13 bis 28 m/g verwenden.

Als inertes Trägermaterial können praktisch alle Trägermaterialien des Standes der Technik, wie sie vorteilhaft bei der Herstellung von Schalenkatalysatoren für die Oxidation aromatischer Kohlenwasserstoffe zu Aldehyden, Carbonsäuren und/oder Carbonsäureanhydriden eingesetzt werden, Verwendung finden, beispielsweise Quarz (SiO₂), Porzellan, Magnesiumoxid, Zinndioxid, Siliciumcarbid, Rutil, Tonerde (Al₂O₃), Aluminiumsilikat, Steatit (Magnesiumsilikat), Zirkoniumsilikat, Cersilikat oder Mischungen dieser Trägermaterialien. Das Trägermaterial ist in der Regel nicht-porös. Der Ausdruck "nicht-porös" ist dabei im Sinne von "bis auf technisch unwirksame Mengen an Poren nicht-porös" zu verstehen, da technisch unvermeidlich eine geringe Anzahl Poren im Trägermaterial, das idealerweise keine Poren enthalten sollte, vorhanden sein können. Als vorteilhafte Trägermaterialien sind insbesondere Steatit und Siliciumcarbid hervorzuheben. Die Form des Trägermaterials ist für die erfindungsgemäßen Präkatalysatoren und Schalenkatalysatoren im Allgemeinen nicht kritisch. Beispielsweise können Katalysatorträger in Form von Kugeln, Ringen, Tabletten, Spiralen, Röhren, Extrudaten oder Splitt verwendet werden. Die Dimensionen dieser Katalysatorträger entsprechen denen üblicherweise zur Herstellung von Schalenkatalysatoren für die Gasphasenpartialoxidation von aromatischen Kohlenwasserstoffen verwendeten Katalysatorträgern. Bevorzugt wird Steatit in Form von Kugeln mit einem Durchmesser von 3 bis 6 mm oder von Ringen mit einem äußeren Durchmesser von 5 bis 9 mm und einer Länge von 3 bis 8 mm und einer Wandstärke von 1 bis 2 mm verwendet.

Die Aufbringung der einzelnen Schichten des Schalenkatalysators kann mit beliebigen an sich bekannten Methoden erfolgen, z. B. durch Aufsprühen von Lösungen oder Suspensionen in der Dragiertrommel oder Beschichtung mit einer Lösung oder Suspension in einer Wirbelschicht.

Bei der Beschichtung des Katalysatorträgers mit der katalytisch aktiven Masse werden im Allgemeinen Beschichtungstemperaturen von 20 bis 500 °C angewandt, wobei die Beschichtung in der Beschichtungsapparatur unter Atmosphärendruck oder unter reduziertem Druck erfolgen kann. Im Allgemeinen erfolgt die Beschichtung bei 0°C bis 200 °C, vorzugsweise bei 20 bis 150 °C, insbesondere bei Raumtemperatur bis 120 °C durchgeführt.

Durch thermische Behandlung des so erhaltenen Präkatalysators bei Temperaturen über 200 bis 500 °C entweicht das Bindemittel durch thermische Zersetzung und/oder Verbrennung aus der aufgetragenen Schicht. Vorzugsweise erfolgt die thermische Behandlung in situ im Gasphasenoxidationsreaktor.

In bevorzugten Ausführungsformen zeichnen sich erfindungsgemäße Katalysatoren nach erfolgter thermische Zersetzung und/oder Verbrennung des Bindemittels (z. B. nach vierstündiger Calzination bei 400 °C) durch einen H₂-Verbrauch von weniger als 5,5 mol/mol Vanadium (mol H₂, bezogen auf die im Katalysator enthaltene Menge Vanadium in mol), vorzugsweise weniger als 5,0 mol/mol Vanadium, aus, wenn sie von 25 auf 923 K in einem Wasserstoff enthaltenden Inertgasstrom erhitzt werden.

Die erfindungsgemäßen Katalysatoren eignen sich generell zur Gasphasenoxidation aromatischer C₆- bis C₁₀-Kohlenwasserstoffe, wie Benzol, den Xylolen, Toluol, Naphthalin oder Durol (1,2,4,5-Tetramethylbenzol) zu Carbonsäuren und/oder Carbonsäureanhydriden wie Maleinsäureanhydrid, Phthalsäureanhydrid, Benzoesäure und/oder Pyromellithsäuredianhydrid.

Insbesondere ermöglichen die neuen Schalenkatalysatoren eine signifikante Steigerung der Selektivität und Ausbeute bei der Herstellung von Phthalsäureanhydrid.

Zu diesem Zweck werden die erfindungsgemäß hergestellten Katalysatoren in von außen auf die Reaktionstemperatur, beispielsweise mittels Salzschmelzen, thermostatisierte Reaktionsrohre gefüllt und über die so bereitete Katalysatorschüttung das Reaktionsgas Temperaturen von im allgemeinen 300 bis 450 °C, vorzugsweise von 320 bis 420 °C und besonders bevorzugt von 340 bis 400 °C und bei einem Überdruck von im allgemeinen 0,1 bis 2,5 bar, vorzugsweise von 0,3 bis 1,5 bar mit einer Raumgeschwindigkeit von im allgemeinen 750 bis 5000 h geleitet.

Das dem Katalysator zugeführte Reaktionsgas wird im allgemeinen durch Vermischen von einem molekularen Sauerstoff enthaltenden Gas, das außer Sauerstoff noch geeignete Reaktionsmoderatoren und/oder Verdünnungsmittel, wie Dampf, Kohlendioxid und/oder Stickstoff, enthalten kann, mit dem zu oxidierenden, aromatischen Kohlenwasserstoff erzeugt, wobei das molekularen Sauerstoff enthaltende Gas im allgemeinen 1 bis 100 mol-%, vorzugsweise 2 bis 50 mol-% und besonders bevorzugt 10 bis 30 mol-% Sauerstoff, 0 bis 30 mol-%, vorzugsweise 0 bis 10 mol-% Wasserdampf sowie 0 bis 50 mol-%, vorzugsweise 0 bis 1 mol-% Kohlendioxid, Rest Stickstoff, enthalten kann. Zur Erzeugung des Reaktionsgases wird das molekularen Sauerstoff enthaltende Gas im allgemeinen mit 30 g bis 150 g je Nm³ Gas des zu oxidierenden, aromatischen Kohlenwasserstoffs beschickt.

Vorteilhaft wird die Gasphasenoxidation so durchgeführt, dass man zwei oder mehr Zonen, vorzugsweise zwei Zonen der im Reaktionsrohr befindlichen Katalysatorschüttung auf unterschiedliche Reaktionstemperaturen thermostatisiert, wozu beispielsweise Reaktoren mit getrennten Salzbädern eingesetzt werden können. Wird die Umsetzung in zwei Reaktionszonen durchgeführt wird im allgemeinen die zum Gaseintritt des Reaktionsgases hin gelegene Reaktionszone, welche im allgemeinen 30 bis 80 mol-% des gesamten Katalysatorvolumens umfasst, auf eine um 1 bis 20 °C, vorzugsweise um 1 bis 10 °C und insbesondere um 2 bis 8 °C höhere Reaktionstemperatur als die Gasaustritt hingelegene Reaktionszone thermostatisiert. Alternativ kann die Gasphasenoxidation auch ohne Aufteilung in Temperaturzonen bei einer Reaktionstemperatur durchgeführt werden.

Unabhängig von der Temperaturstrukturierung hat es sich als besonders vorteilhaft erwiesen, wenn in den oben angegebenen Reaktionszonen der Katalysatorschüttung Katalysatoren eingesetzt werden, die sich in ihrer katalytischen Aktivität und/oder chemischen Zusammensetzung ihrer Aktivmasse unterscheiden. Vorzugsweise wird bei Anwendung zweier Reaktionszonen in der ersten, also zum Gaseintritt des Reaktionsgases hin gelegenen Reaktionszone, ein Katalysator eingesetzt, der in Vergleich zum Katalysator, welcher sich in der zweiten, also zum Gasaustritt hin gelegenen Reaktionszone, befindet, eine etwas geringere katalytische Aktivität hat. Im allgemeinen wird die Umsetzung durch die Temperatureinstellung so gesteuert, dass in der ersten Zone der größte Teil der im Reaktionsgas enthaltenen aromatischen Kohlenwasserstoff bei maximaler Ausbeute umgesetzt wird.

Bevorzugt werden drei- bis fünflagige Katalysatorsysteme verwendet, insbesondere drei- und vierlagige Katalysatorsysteme.

In einer bevorzugten Ausführungsform eines dreilagigen Katalysatorsystems weisen die Katalysatoren folgende Zusammensetzung auf:
- für die erste, oberste Schicht (Schicht a)):
   7 bis 10 Gew.-% Aktivmasse bezogen auf den gesamten Katalysator, wobei diese Aktivmasse:
      6 bis 11 Gew.-% Vanadium (berechnet als V₂O₅)
      0 bis 3 Gew.-% Antimontrioxid
      0,1 bis 1 Gew.-% eines Alkali (ber. als Alkalimetall), insbesondere Cäsiumoxid enthält und als Rest zu 100 Gew.-% Titandioxid in Anatasmodifikation mit einer BET-Oberfläche von 5 bis 15 m²/g aufweist
- für die zweite, mittlere Schicht (Schicht b)):
   7 bis 12 Gew.-% Aktivmasse bezogen auf den gesamten Katalysator, wobei diese Aktivmasse:
      5 bis 13 Gew.-% Vanadium (berechnet als V₂O₅)
      0 bis 3 Gew.-% Antimontrioxid
      0 bis 0,4 Gew.-% eines Alkali (ber. als Alkalimetall), insbesondere Cäsiumoxid
      0 bis 0,4 Gew.-% Phosphorpentoxid (berechnet als P)
      enthält und als Rest zu 100 Gew.-% Titandioxid in Anatasmodifikation, gegebenenfalls wie in Schicht a)
- für die dritte, unterste Schicht (Schicht c)):
   8 bis 12 Gew.-% Aktivmasse bezogen auf den gesamten Katalysator, wobei diese Aktivmasse:
      5 bis 30 Gew.-% Vanadium (berechnet als V₂O₅)
      0 bis 3 Gew.-% Antimontrioxid
      0 bis 0,3 Gew.-% eines Alkali (ber. als Alkalimetall), insbesondere Cäsiumoxid
      0,05 bis 0,4 Gew.-% Phosphorpentoxid (berechnet als P)
      enthält und als Rest zu 100 Gew.-% Titandioxid, insbesondere in Anatasmodifikation, gegebenenfalls wie in Schicht a).

In einer bevorzugten Ausführungsform eines vierlagigen Katalysatorsystems weisen die Katalysatoren folgende Zusammensetzung auf:
- für die erste Schicht (Schicht a)):
   7 bis 10 Gew.-% Aktivmasse bezogen auf den gesamten Katalysator, wobei diese Aktivmasse:
      6 bis 11 Gew.-% Vanadium (berechnet als V₂O₅)
      0 bis 3 Gew.-% Antimontrioxid
      0,1 bis 1 Gew.-% eines Alkali (ber. als Alkalimetall), insbesondere Cäsiumoxid enthält und als Rest zu 100 Gew.-% Titandioxid in Anatasmodifikation mit einer BET-Oberfläche von 5 bis 15 m²/g aufweist
- für die zweite Schicht (Schicht b1)):
   7 bis 12 Gew.-% Aktivmasse bezogen auf den gesamten Katalysator, wobei diese Aktivmasse:
      4 bis 15 Gew.-% Vanadium (berechnet als V₂O₅)
      0 bis 3 Gew.-% Antimontrioxid
      0,1 bis 1 Gew.-% eines Alkali (ber. als Alkalimetall), insbesondere Cäsiumoxid
      0 bis 0,4 Gew.-% Phosphorpentoxid (berechnet als P)
      enthält und als Rest zu 100 Gew.-% Titandioxid in Anatasmodifikation, gegebenenfalls wie in Schicht a)
- für die dritte Schicht (Schicht b2)):
   7 bis 12 Gew.-% Aktivmasse bezogen auf den gesamten Katalysator, wobei diese Aktivmasse:
      5 bis 15 Gew.-% Vanadium (berechnet als V₂O₅)
      0 bis 3 Gew.-% Antimontrioxid
      0 bis 0,4 Gew.-% eines Alkali (ber. als Alkalimetall), insbesondere Cäsiumoxid
      0 bis 0,4 Gew.-% Phosphorpentoxid (berechnet als P)
      enthält und als Rest zu 100 Gew.-% Titandioxid in Anatasmodifikation, gegebenenfalls wie in Schicht a)
- für die vierte Schicht (Schicht c)):
   8 bis 12 Gew.-% Aktivmasse bezogen auf den gesamten Katalysator, wobei diese Aktivmasse:
      5 bis 30 Gew.-% Vanadium (berechnet als V₂O₅)
      0 bis 3 Gew.-% Antimontrioxid
      0,05 bis 0,4 Gew.-% Phosphorpentoxid (berechnet als P)
      enthält und als Rest zu 100 Gew.-% Titandioxid in Anatasmodifikation, gegebenenfalls wie in Schicht a).

Allgemein können die Katalysatorschichten a), b), c) und/oder d) auch so angeordnet sein, dass sie jeweils aus zwei oder mehreren Schichten bestehen. Diese Zwischenschichten haben vorteilhaft intermediate Katalysatorzusammensetzungen.

Anstelle von gegeneinander abgegrenzter Schichten der verschiedenen Katalysatoren kann auch ein quasi-kontinuierlicher Übergang der Schichten und ein quasigleichmässiger Anstieg der Aktivität dadurch bewirkt werden, dass man beim Übergang von einer Schicht zur nächsten Schicht eine Zone mit einer Vermischung der aufeinander folgenden Katalysatoren vornimmt.

Die Schüttungslänge der ersten Katalysatorschicht macht vorzugsweise mehr als 30 bis 80 % der gesamten Katalysatorfüllhöhe im Reaktor aus. Die Schüttungshöhe der ersten beiden, bzw. der ersten drei Katalysatorschichten macht vorteilhaft mehr als 60 bis 95 % der gesamten Katalysatorfüllhöhe aus. Typische Reaktoren weisen eine Füllhöhe von 250 cm bis 350 cm auf. Die Katalysatorschichten können auch gegebenenfalls auf mehrere Reaktoren verteilt werden.

Gewünschtenfalls kann man für die Phthalsäureanhydridherstellung noch einen nachgeschalteten Finishing-Reaktor vorsehen, wie er beispielsweise in der DE-A 198 07 018 oder DE-A 20 05 969 beschrieben ist. Als Katalysator verwendet man dabei im Vergleich zum Katalysator der letzten Schicht vorzugsweise einen noch aktiveren Katalysator.

Wird die PSA-Herstellung mit den erfindungsgemäßen Katalysatoren unter Anwendung mehrerer Reaktionszonen, in denen sich unterschiedliche Katalysatoren befinden, durchgeführt, so können in allen Reaktionszonen die neuen Schalenkatalysatoren eingesetzt werden. Es können aber im allgemeinen bereits erhebliche Vorteile gegenüber herkömmlichen Verfahren erzielt werden, wenn nur in einer der Reaktionszonen der Katalysatorschüttung, beispielsweise der ersten Reaktionszone, oder den ersten beiden Reaktionszonen, erfindungsgemäße Schalenkatalysator verwendet werden und in den übrigen Reaktionszonen auf herkömmliche Weise hergestellte Schalenkatalysatoren benutzt werden. In der (den) ersten Reaktionszone(n) herrschen im Vergleich zu den stromabwärts gelegenen Reaktionszonen höhere Hotspot-Temperaturen; hier wird der Hauptteil des Ausgangskohlenwasserstoffs zu dem gewünschten Oxidationsprodukt und/oder Intermediaten oxidiert, so dass die Vorteile der erfindungsgemäßen Katalysatore in der ersten Stufe oder der ersten und zweiten Stufe besonders zum Tragen kommen.

Die Erfindung wird durch die folgenden Beispiele näher veranschaulicht.

### A. Katalysatorherstellung

### Oberschichtkatalysatoren R1.1 bis R1.4

34,64 g Titandioxid (BET-Oberfläche 9 m2/g), 64,33 g Titandioxid (BET-Oberfläche 20 m2/g), 7,82 g Vanadiumpentoxid, 2,60 g Antimonoxid, 0,444 g Cäsiumcarbonat wurden in 650 ml entionisiertem Wasser suspendiert und 18 Stunden gerührt. Dieser Suspension wurden die in der nachstehenden Tabelle 1 angegebenen Binder zugesetzt. Die erhaltene Suspension wurde anschließend auf 1200 g Steatit (Magnesiumsilikat) in Form von Ringen (7 x 7 x 4 mm, Außendurchmesser, Länge, Innendurchmesser) aufgesprüht und getrocknet. Das Gewicht der aufgebrachten Schale betrug 8,0 % des Gesamtgewichts des fertigen Katalysators. Die auf diese Weise aufgebrachte katalytisch aktive Masse enthielt nach vierstündiger Calcination bei 400 °C 7,12 Gew.% Vanadium (berechnet als V₂O₅), 2,37 Gew.-% Antimon (berechnet als Sb₂O₃), 0,33 Gew.-% Cäsium (berechnet als Cs) und 90,1 Gew.-% Titandioxid.

**Tabelle 1**

| Kat. | Binder (molares Comonomerverhältnis) | Bindermenge [g] |
|---|---|---|
| R1.1 | Vinylacetat-Ethylen-Copolymer (63:37) | 25 |
| R1.2 | Vinylacetat-Ethylen-Copolymer (67:33) | 25 |
| R1.3* | Vinylacetat-Ethylen-Copolymer (60:40) | 25 |
| R1.4* | Hydroxyethylcellulose | 4 |

| | | |
|---|---|---|
| *Vergleichsbeispiele | | |

### Mittelschichtkatalysator R2:

34,32 g Titandioxid (BET-Oberfläche 9 m²/g), 102,90 g Titandioxid (BET-Oberfläche 20 m²/g), 11,0 g Vanadiumpentoxid, 2,30 g Ammoniumdihydrogenphosphat, 3,66 g Antimonoxid, 0,19 g Cäsiumcarbonat wurden in 650 ml Wasser suspendiert und 18 Stunden gerührt. Dieser Suspension wurden 50 g organischer Binder, bestehend aus einem Copolymer aus Vinylacetat und Vinyllaurat, in Form einer 50 Gew.-%igen Dispersion zugegeben. Die erhaltene Suspension wurde anschließend auf 1200 g Steatit (Magnesiumsilikat) in Form von Ringen (7 x 7 x 4 mm, Außendurchmesser, Länge, Innendurchmesser) aufgesprüht und getrocknet. Das Gewicht der aufgebrachten Schale betrug 10,0 % des Gesamtgewichts des fertigen Katalysators. Die auf diese Weise aufgebrachte katalytisch aktive Masse enthielt nach vierstündiger Calcination bei 400 °C 7,12 Gew.% Vanadium (berechnet als V₂O₅), 0,40 Gew.-% Phosphor (berechnet als P), 2,37 Gew.-% Antimon (berechnet als Sb₂O₃), 0,10 Gew.-% Cäsium (berechnet als Cs) und 88,91 Gew.-% Titandioxid.

### Unterschichtkatalysator R3:

24,56 g Titandioxid (BET-Oberfläche 9 m²/g), 73,67 g Titandioxid (BET-Oberfläche 30 m²/g), 24,99 g Vanadiumpentoxid und 1,71 g Ammoniumdihydrogenphosphat wurden in 650 ml Wasser suspendiert und 18 Stunden gerührt. Dieser Suspension wurden 58,6 g organischer Binder, bestehend aus einem Copolymer aus Vinylacetat und Ethylen (Molverhältnis 63:37), in Form einer 50 Gew.-%igen Dispersion zugegeben. Die erhaltene Suspension wurde anschließend auf 1200 g Steatit (Magnesiumsilikat) in Form von Ringen (7 x 7 x 4 mm, Außendurchmesser, Länge, Innendurchmesser) aufgesprüht und getrocknet. Das Gewicht der aufgebrachten Schale betrug 9,3 % des Gesamtgewichts des fertigen Katalysators. Die auf diese Weise aufgebrachte katalytisch aktive Masse enthielt nach vierstündiger Calcination bei 400 °C 19,81 Gew.% Vanadium (berechnet als V₂O₅), 0,45 Gew.-% Phosphor (berechnet als P) und 78,63 Gew.-% Titandioxid.

### B. Katalysatortest der Katalysatoren R1.1 bis R1.4 mittels temperaturprogrammierter Reduktion (TPR)

Die temperaturprogrammierte Reduktion erfolgte durch Aufheizen der Probe in einem Wasserstoff/Inertgas-Strom mit konstanter Temperaturerhöhung pro Zeiteinheit. Man verwendete eine Anordnung, deren Aufbau auf den Vorschlägen von Monti und Baiker [D.A.M. Monti, A. Baiker, "Temperatur-Programmed Reduction. Parametric Sensitivity and Estimation of Kinetic Parameters", J. Catal. 83 (1983) 323- 335] beruht. Die Proben wurden als lose Schüttung zwischen zwei Glaswollepropfen in ein U-förmiges Glasrohr eingefüllt. Das U-Rohr befand sich in einem Röhrenofen aus Keramik. Zuerst wurden die Katalysator unter Durchleiten von Luft (Sauerstoffüberschuss) 4 Stunden bei 400 °C calziniert.

Nach dem Abkühlen wurde die Probe mit einer Heizrampe von 10 K/min von Umgebungstemperatur auf eine Endtemperatur von 923 K aufgeheizt. Die Probentemperatur wurde in einer Thermoelementhülse nahe der Schüttung gemessen und in Intervallen von 2 s aufgezeichnet. Durch das U-Rohr wurde ein Wasserstoff/Argon-Strom mit 4,2 % Wasserstoff geleitet. Der Wasserstoffgehalt im Abgas wurde mit einem Wärmeleitfähigkeitsdetektor bestimmt. Zur Kalibrierung des Wärmeleitfähigkeitsdetektors diente die Reduktion von CuO zu Cu(0). Der Wasserstoffverbrauch wurde in Abhängigkeit von der Temperatur aufgezeichnet. Durch Integration wurde der gesamte H₂-Verbrauch im untersuchten Temperaturintervall ermittelt.

**Tabelle 2**

| Kat. | H₂-Verbrauch [mmol/g] | H₂-Verbrauch [mol/mol V] |
|---|---|---|
| R1.1 | 0,89 | 4,6 |
| R1.2 | 0,89 | 4,6 |
| R1.3* | 1,31 | 6,7 |
| R1.4* | 1,17 | 6 |

| | | |
|---|---|---|
| *Vergleichsbeispiele | | |

### C. Phthalsäureanhydrid-Herstellung (strukturierte Schüttung mit zwei Katalysatorlagen aus R1 und R2)

Von unten nach oben wurden jeweils 1,30 m des Katalysators R2 und 1,50 m des Katalysators R1.1 bzw. R1.4 in ein 3,3 m langes Eisenrohr mit einer lichten Weite von 25 mm eingefüllt. Das Eisenrohr war zur Temperaturregelung von einer Salzschmelze umgeben, eine 2 mm Thermohülse mit eingebautem Zugelement diente der Katalysatortemperaturmessung. Durch das Rohr wurden stündlich von oben nach unten 4,0 Nm³ Luft mit einer Beladung von etwa 40 g/Nm³ 99,3 Gew.-%igem o-Xylol gleitet. Es wurden die in der nachstehenden Tabelle 3 zusammengestellten Ergebnisse erhalten. "PSA-Ausbeute" bedeutet das erhaltene Phthalsäureanhydrid in Gewichtsteilen, bezogen auf 100 Gewichtteile reines o-Xylol.

**Tabelle 3**

| Kat. | R1.1/R2 | R1.4/R2 |
|---|---|---|
| Beladung [g/Nm3] | 46 | 37 |
| Salzbadtemperatur [°C] | 400 | 400 |
| Hotspot-Temperatur Oberschicht [°C] | 464 | 432 |
| Durchschnittliche PSA-Ausbeute | 106,6 | 104,3 |
| Rest o-Xylol [Gew.-%] | 0,01 | 0,01 |
| Phthalid [Gew.-%] | 0,11 | 0,80 |

Man erkennt, dass unter Verwendung des erfindungsgemäßen Katalysators R1.1 eine höhere PSA-Ausbeute und bessere Produktqualität erzielt werden.

### D. Phthalsäureanhydrid-Herstellung (strukturierte Schüttung mit drei Katalysatorlagen aus R1, R2 und R3)

Von unten nach oben wurden jeweils 0,70 m des Katalysators R3, 0,60 m des Katalysators R2 und 1,50 m des Katalysators R1.1 in ein 3,85 m langes Eisenrohr mit einer lichten Weite von 25 mm eingefüllt. Das Eisenrohr war zur Temperaturregelung von einer Salzschmelze umgeben, eine 2 mm Thermohülse mit eingebautem Zugelement diente der Katalysatortemperaturmessung. Durch das Rohr wurden stündlich von oben nach unten 4,0 Nm³ Luft mit einer Beladung von etwa 70 g/Nm³ 99,3 Gew.-%igem o-Xylol gleitet. Es wurden die in der nachstehenden Tabelle 4 zusammengestellten Ergebnisse erhalten.

**Tabelle 4**

| Kat. | R1.1/R2/R3 |
|---|---|
| Beladung [g/Nm3] | 70 |
| Salzbadtemperatur [°C] | 365 |
| Hotspot-Temperatur Oberschicht [°C] | 432 |
| Durchschnittliche PSA-Ausbeute | 112,5 |
| Rest o-Xylol [Gew.-%] | < 0,01 |
| Phthalid [Gew.-%] | 0,01 |

## Patentansprüche

1. Katalysator für Gasphasenoxidationen, umfassend einen inerten Träger und eine darauf aufgebrachte, Übergangsmetalloxide enthaltende katalytisch aktive Masse, oder Präkatalysator dafür, erhältlich durch Behandeln des inerten Trägers mit einer wässrigen Suspension oder Lösung der Übergangsmetalloxide oder deren Vorläuferverbindungen, wobei die Suspension eine Bindemitteldispersion enthält und das Bindemittel ein Copolymer eines α-Olefins und eines Vinyl-C₂-C₄-carboxylats ist, dessen Vinyl-C₂-C₄-carboxylatgehalt wenigstens 62 Mol-% beträgt.

2. Katalysator nach Anspruch 1, wobei das Vinyl-C₂-C₄-carboxylatcopolymer ein Vinylacetatcopolymer ist.

3. Katalysator nach Anspruch 2, wobei das Vinylacetatcopolymer ein Ethylen-Vinylacetat-Copolymer ist.

4. Katalysator nach Anspruch 3, wobei das Ethylen-Vinylacetat-Copolymer 63 bis 70 Mol-% Vinylacetat und 37 bis 30 Mol-% Ethylen enthält.

5. Katalysator nach einem der vorhergehenden Ansprüche, wobei die katalytisch aktive Masse, bezogen auf die Gesamtmenge der katalytisch aktiven Masse, 1 bis 40 Gew.-% Vanadiumoxid, berechnet als V₂O₅, 60 bis 99 Gew.-% Titandoxid, berechnet als TiO₂.enthält.

6. Katalysator nach Anspruch 5, wobei die katalytisch aktive Masse, bezogen auf die Gesamtmenge der katalytisch aktiven Masse, bis zu 1 Gew.-% einer Cäsiumverbindung, berechnet als Cs, bis zu 1 Gew.-% einer Phosphorverbindung, berechnet als P und bis zu 10 Gew.-% Antimonoxid, berechnet als Sb₂O₃ enthält.

7. Verfahren zur Herstellung von Aldehyden, Carbonsäuren und/oder Carbonsäureanhydriden, bei dem man einen gasförmigen Strom, der einen aromatischen Kohlenwasserstoff und ein molekularen Sauerstoff enthaltendes Gas umfasst, bei erhöhter Temperatur mit einem Katalysator nach einem der Ansprüche 1 bis 6 in Kontakt bringt.

8. Verfahren nach Anspruch 7, bei dem man den Katalysator in situ aus einem Präkatalysator erzeugt.

9. Verfahren nach Anspruch 7 oder 8, bei dem man als aromatischen Kohlenwasserstoff o-Xylol oder Naphthalin oder Mischungen aus o-Xylol und Naphthalin zu Phthalsäureanhydrid oxidiert.

## Claims

1. A catalyst for gas-phase oxidations, comprising an inert support and a catalytically active composition comprising transition metal oxides applied thereto, or a precatalyst for this, obtainable by treating' an inert support with an aqueous suspension or solution of the transition metal oxides or their precursor compounds, wherein the suspension comprises a binder dispersion and the binder is a copolymer of an α-olefin and a vinyl-C₂-C₄-carboxylate whose vinyl C₂-C₄-carboxylate content is at least 62 mol%.

2. The catalyst according to claim 1, wherein the vinyl C₂-C₄-carboxylate copolymer is a vinyl acetate copolymer.

3. The catalyst according to claim 2, wherein the vinyl acetate copolymer is an ethylene-vinyl acetate copolymer.

4. The catalyst according to claim 3, wherein the ethylene-vinyl acetate copolymer comprises from 63 to 70 mol% of vinyl acetate and from 37 to 30 mol% of ethylene.

5. The catalyst according to any of the preceding claims, wherein the catalytically active composition comprises from 1 to 40% by weight of vanadium oxide, calculated as V₂O₅, and from 60 to 99% by weight of titanium dioxide, calculated as TiO₂., based on the total amount of the catalytically active composition.

6. The catalyst according to claim 5, wherein the catalytically active composition further comprises up to 1% by weight of a cesium compound, calculated as Cs, up to 1% by weight of a phosphorus compound, calculated as P, and up to 10% by weight of antimony oxide, calculated as Sb₂O₃, based on the total amount of the catalytically active composition.

7. A process for preparing aldehydes, carboxylic acids and/or carboxylic anhydrides, which comprises bringing a gaseous stream comprising an aromatic hydrocarbon and a gas comprising molecular oxygen into contact with a catalyst according to any of claims 1 to 6 at elevated temperature.

8. The process according to claim 7, wherein the catalyst is produced in situ from a precatalyst.

9. The process according to claim 7 or 8, wherein o-xylene or naphthalene or a mixture of o-xylene and naphthalene is used as aromatic hydrocarbon and is oxidized to phthalic anhydride.

## Revendications

1. Catalyseur pour oxydations en phase gazeuse, comprenant un support inerte et, appliquée sur celui-ci, une masse active au plan catalytique, qui contient des oxydes de métaux de transition, ou un pré-catalyseur à cet effet, que l'on peut obtenir par traitement du support inerte avec une suspension ou une solution aqueuse des oxydes de métaux de transition ou de leurs composés précurseurs, la suspension contenant une dispersion de liant et le liant étant un copolymère d'une α-oléfine et d'un carboxylate de vinyle en C₂-C₄, dont la teneur en carboxylate de vinyle en C₂-C₄ est d'au moins 62% en mole.

2. Catalyseur selon la revendication 1, dans lequel le copolymère de carboxylate de vinyle en C₂-C₄ est un copolymère d'acétate de vinyle.

3. Catalyseur selon la revendication 2, dans lequel le copolymère d'acétate de vinyle est un copolymère de type éthylène-acétate de vinyle.

4. Catalyseur selon la revendication 3, dans lequel le copolymère de type éthylène-acétate de vinyle contient 63 à 70% en mole d'acétate de vinyle et 37 à 30% en mole d'éthylène.

5. Catalyseur selon l'une quelconque des revendications précédentes, dans lequel la masse active au plan catalytique contient, par rapport à la quantité totale de masse active au plan catalytique, 1 à 40% en poids d'oxyde de vanadium, calculé comme V₂O₅, 60 à 99% en poids de dioxyde de titane, calculé comme TiO₂.

6. Catalyseur selon la revendication 5, dans lequel la masse active au plan catalytique contient, par rapport à la quantité totale de masse active au plan catalytique, jusqu'à 1% en poids d'un composé de césium, calculé comme Cs, jusqu'à 1% en poids d'un composé de phosphore, calculé comme P et jusqu'à 10% en poids d'oxyde d'antimoine, calculé comme Sb₂O₃.

7. Procédé de fabrication d'aldéhydes, d'acides carboxyliques et/ou d'anhydrides d'acides carboxyliques, dans lequel on met en contact à température élevée un courant gazeux, qui comprend un gaz contenant un hydrocarbure aromatique et de l'oxygène moléculaire, avec un catalyseur selon l'une quelconque des revendications 1 à 6.

8. Procédé selon la revendication 7, dans lequel on produit le catalyseur in situ à partir d'un pré-catalyseur.

9. Procédé selon la revendication 7 ou 8, dans lequel on effectue l'oxydation de o-xylène ou de naphtalène ou de mélanges de o-xylène et de naphtalène, comme hydrocarbures aromatiques, pour former de l'anhydride d'acide phtalique.
